# EUROPÄISCHE PATENTANMELDUNG

(11) **EP 4 039 885 A1**
(43) Veröffentlichungstag der Anmeldung: **10.08.2022**
(21) Anmeldenummer: 21155759.0
(22) Anmeldetag: 08.02.2021
(51) Int. Cl.: E02D 5/34, G01K 11/32, G01K 1/143, G01N 33/38, E04C 5/01, G01M 11/08

(54) **VERFAHREN UND VORRICHTUNG ZUR THERMISCHEN ÜBERWACHUNG EINER STRUKTUR AUS EINEM UNTER TEMPERATURÄNDERUNG ABBINDENDEN STOFF**

(71) Anmelder: Züblin Spezialtiefbau GmbH, 70567 Stuttgart (DE); GEO-Inspector Bruns Kuhn Ingenieure GbR, 38114 Braunschweig (DE)
(72) Erfinder: BRUNS, Benedikt, 31139 Hildesheim (DE); KUHN, Christian, 38114 Braunschweig (DE); ZECH, Robert, 14050 Berlin (DE); PERL, Christian, 04654 Frohburg (DE)
(74) Vertreter: Liedtke & Partner Patentanwälte

(57) **Zusammenfassung**

Die Erfindung betrifft ein Verfahren und eine Vorrichtung zur thermischen Überwachung einer Struktur aus einem unter Temperaturänderung abbindenden Stoff, wobei:
- eine Hilfskonstruktion (1) und/oder eine tragende Konstruktion mit mindestens einem daran angeordneten Hüllrohr (2) installiert wird,
- mindestens ein Glasfasermesssensor (4), dessen eines Ende mit einem Abfragegerät (3) verbunden ist oder wird und dessen anderes Ende offen ist, in das mindestens eine Hüllrohr (2) eingebracht wird, derart, dass das offene Ende sich im Hüllrohr (2) befindet,
- der unter Temperaturänderung abbindende Stoff in die Hilfskonstruktion (1) und/oder tragende Konstruktion eingebracht wird, und
- vom Abfragegerät (3) mittels faseroptischer Temperaturmessung eine Temperaturentwicklung entlang des Glasfasermesssensors (4) während des Abbindens erfasst wird.

## Beschreibung

Die Erfindung betrifft ein Verfahren und eine Vorrichtung zur thermischen Überwachung einer Struktur aus einem unter Temperaturänderung, meist Wärmeabgabe, abbindenden Stoff.

Herkömmliche Verfahren zur Integritätsprüfung von Betonpfählen erfolgen nach der Erhärtung des Betons und weisen Einschränkungen auf, beispielsweise bei der Bewertung der Betonqualität im unteren Pfahlsegment bei großer Pfahllänge (Hammerschlagmethode) oder im Pfahlquerschnitt außerhalb des Bewehrungskorbes (Ultraschallmessung). Beim Thermal Integrity Profiling (TIP) wird die Verteilung der Abbindewärme beim Aushärten des Betons genutzt, um die Betonqualität über den gesamten Querschnitt und über die gesamte Pfahllänge frühzeitig zu bewerten. Eine gleichmäßige Temperaturverteilung zeigt eine gleichmäßige Umschließung des Sensors mit Beton an. Niedrigere Temperaturen sind Hinweise auf z. B. Einschlüsse, Risse oder geringere Betonqualität. Zur Messung der Abbindewärme über die Pfahllänge ist die Verwendung faseroptischer Temperatursensoren beispielsweise aus EP 3 102 937 B1 bekannt.

Der Erfindung liegt die Aufgabe zu Grunde, ein verbessertes Verfahren und eine verbesserte Vorrichtung zur thermischen Überwachung einer Struktur aus einem unter Temperaturänderung, in den allermeisten Fällen Wärmeabgabe, abbindenden Stoff anzugeben.

Die Aufgabe wird erfindungsgemäß gelöst durch ein Verfahren zur thermischen Überwachung einer Struktur aus einem unter Temperaturänderung abbindenden Stoff gemäß Anspruch 1 und durch eine Vorrichtung zur thermischen Überwachung einer Struktur aus einem unter Temperaturänderung abbindenden Stoff gemäß Anspruch 10.

Vorteilhafte Ausgestaltungen der Erfindung sind Gegenstand der Unteransprüche.

Bei einem erfindungsgemäßen Verfahren zur thermischen Überwachung einer Struktur aus einem unter Temperaturänderung, beispielsweise Wärmeabgabe oder Wärmeaufnahme, abbindenden Stoff wird eine Hilfskonstruktion und/oder eine tragende Konstruktion, beispielsweise ein Armierungskorb oder -käfig für einen Pfahl, mit mindestens einem daran angeordneten Hüllrohr installiert, beispielsweise in einem Baugrund. Weiter wird mindestens ein Glasfasermesssensor, dessen eines Ende mit einem Abfragegerät verbunden ist oder wird und dessen anderes Ende offen ist, in das mindestens eine Hüllrohr eingebracht, derart, dass sich das offene Ende im Hüllrohr befindet. Weiter wird der unter Temperaturänderung abbindende Stoff in die Hilfskonstruktion und/oder tragende Konstruktion eingebracht, um die Struktur zu bilden. Anschließend wird vom Abfragegerät mittels faseroptischer Temperaturmessung eine Temperaturentwicklung entlang des Glasfasermesssensors während des Abbindens erfasst.

Insbesondere wird der Glasfasermesssensor ins Hüllrohr so eingebracht, dass er nicht als Schleife ausgebildet oder gefaltet oder auf sich selbst gedoppelt ist, sondern einfach entlang des Hüllrohrs verläuft, beispielsweise gerade. Ebenso kann das Hüllrohr Biegungen aufweisen, denen der Glasfasermesssensor folgt.

In einer Ausführungsform erfolgt im Abfragegerät ferner eine Analyse der Temperaturentwicklung zur Bestimmung der Homogenität des unter Temperaturänderung abbindenden Stoffes.

In einer Ausführungsform kann der mindestens eine Glasfasermesssensor nach Beendigung der Temperaturmessung aus dem mindestens einen Hüllrohr gezogen werden. Anschließend ist eine Wiederverwendung des Glasfasermesssensors möglich.

In einer Ausführungsform wird als unter Temperaturänderung abbindender Stoff ein hydraulisch abbindender Stoff oder ein Polymer verwendet. Der hydraulisch abbindende Stoff kann zur Herstellung einer Betonstruktur verwendet werden. Eine Betonstruktur ist eine Struktur, die unter Verwendung hydraulisch erhärtender Bindemittel in Verbindung mit Zuschlagstoffen und/oder Bodenmaterial hergestellt wird. Die Betonstruktur kann beispielsweise eine unterirdische Betonstruktur sein, insbesondere ein Bohrpfahl, eine Schlitzwand oder ein Fundamentkörper. Ebenso kann die Betonstruktur eine oberirdische Betonstruktur sein.

In einer Ausführungsform werden mindestens zwei Hüllrohre so angeordnet, dass sie untereinander Kontakt haben oder voneinander beabstandet sind.

In einer Ausführungsform wird der mindestens eine Glasfasermesssensor vor, nach oder während des Einbringens des unter Temperaturänderung abbindenden Stoffes zur Herstellung der Struktur in das offene Ende des mindestens einen Hüllrohrs eingebracht.

In einer Ausführungsform wird der mindestens eine Glasfasermesssensor in das mindestens eine Hüllrohr eingeschoben oder eingeblasen.

In einer Ausführungsform wird der mindestens eine Glasfasermesssensor über die ganze Länge des Hüllrohrs bis zu dessen geschlossenem Ende eingebracht.

In einer Ausführungsform kann mehr als ein Glasfasermesssensor pro Hüllrohr vorgesehen sein. Mehr als ein Glasfasermesssensor pro Hüllrohr erhöht die Messgenauigkeit durch Ausmitteln der Messergebnisse an nebeneinanderliegenden Stellen der Glasfasermesssensoren. Der Glasfasermesssensor kann hierzu auch unten umgebogen sein und an sich selbst entlang im Hüllrohr zurücklaufen.

In einer Ausführungsform wird das mindestens eine Hüllrohr, in dem der Glasfasermesssensor bereits angeordnet ist, vor dem Einbringen des unter Wärmeabgabe abbindenden Stoffes mit einer Flüssigkeit oder mit einem Gel oder mit einer Suspension befüllt.

Die Temperaturmessung kann in Echtzeit erfolgen.

Als Glasfasermesssensor kann eine Single-Mode-Glasfaser oder eine MultiMode-Glasfaser verwendet werden.

Gemäß einem Aspekt der vorliegenden Erfindung wird eine Vorrichtung zur thermischen Überwachung einer Struktur aus einem unter Temperaturänderung, beispielsweise Wärmeabgabe oder Wärmeaufnahme, abbindenden Stoff vorgeschlagen, wobei die thermische Überwachung insbesondere mittels des oben beschriebenen Verfahrens erfolgen kann. Die Vorrichtung umfasst:
- mindestens ein Hüllrohr, das an einer Hilfskonstruktion und/oder an einer tragenden Konstruktion angeordnet oder anordbar ist,
- mindestens ein Abfragegerät,
- mindestens einen Glasfasermesssensor, dessen eines Ende mit dem mindestens einen Abfragegerät verbunden oder verbindbar ist und dessen anderes Ende offen ist,
- wobei das Abfragegerät zur faseroptischen Temperaturmessung einer Temperaturentwicklung entlang des Glasfasermesssensors konfiguriert ist.

In einer Ausführungsform ist das mindestens eine Hüllrohr an einem Ende offen und an einem anderen Ende verschlossen.

In einer Ausführungsform ist das mindestens eine Hüllrohr perforiert oder seitlich geschlitzt.

In einer Ausführungsform ist das mindestens eine Hüllrohr seitlich geschlitzt, so dass der Glasfasermesssensor auch seitlich in das Hüllrohr eingedrückt werden kann. Vorzugsweise ist der Schlitz etwas schmaler als die Dicke des Glasfasermesssensors, so dass dieser, einmal hineingedrückt, seitlich nicht mehr herausrutscht. Ist das Hüllrohr elastisch genug, so können die Ränder des Schlitzes auch geschlossen aneinander liegen. Auf diese Weise bleibt das Innere des Hüllrohrs frei von Beton und kann auch mit einer Flüssigkeit, einem Gel oder einer Suspension gefüllt werden.

Ausführungsbeispiele der Erfindung werden im Folgenden anhand von Zeichnungen näher erläutert.

Darin zeigen:
- Figur 1: eine schematische Ansicht einer Hilfskonstruktion für eine Betonstruktur, einer Anzahl in der Hilfskonstruktion angeordneter Hüllrohre, eines Abfragegerät und mehrerer Glasfasermesssensoren, und
- Figur 2: eine schematische Ansicht der Hilfskonstruktion, der Hüllrohre, des Abfragegeräts und der Glasfasermesssensoren, die jeweils in eines der Hüllrohre eingeschoben sind.

Einander entsprechende Teile sind in allen Figuren mit den gleichen Bezugszeichen versehen.

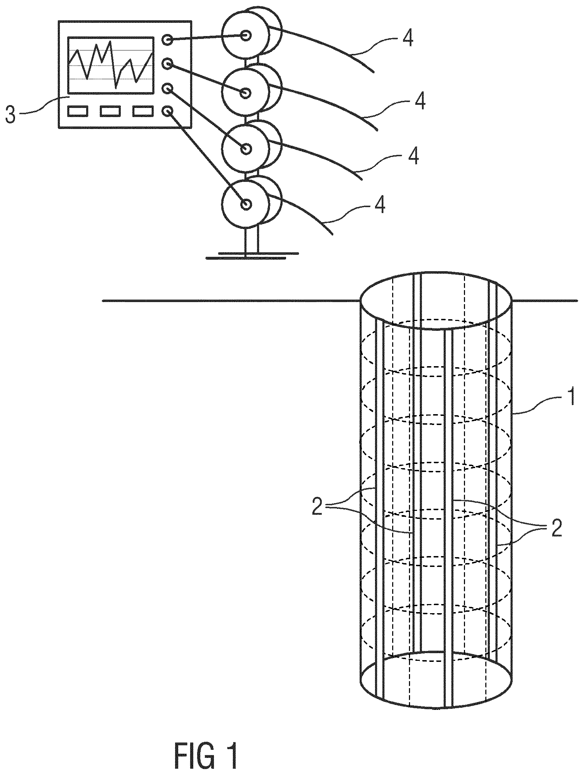

Figur 1 ist eine schematische Ansicht einer Hilfskonstruktion 1 für eine Betonstruktur, wobei mindestens ein Hüllrohr 2, vorzugsweise mehrere Hüllrohre 2, in der Hilfskonstruktion 1 angeordnet sind. Ferner ist ein Abfragegerät 3 vorgesehen, das mit einem Ende mindestens eines, vorzugsweise mehrerer, Glasfasermesssensoren 4 verbunden ist. Das andere Ende des mindestens einen Glasfasermesssensors 4 ist offen.

Die Hilfskonstruktion 1 kann beispielsweise korbartig aus Armierungsstahl gebildet sein.

Ebenso können mehrere Abfragegeräte 3 vorgesehen sein, die jeweils mit mindestens einem Glasfasermesssensor 4 verbunden sind. Der mindestens eine Glasfasermesssensor 4 kann eine beliebige Länge aufweisen.

Mittels des Abfragegeräts 3 und des mindestens einen Glasfasermesssensors 4 ist es möglich, eine Temperaturentwicklung entlang des Glasfasermesssensors 4 zu erfassen und dadurch Stetigkeiten und Unstetigkeiten in der Betonstruktur zu dokumentieren.

Zur Herstellung der Betonstruktur wird das mindestens eine Hüllrohr 2 installiert, das von einem hydraulisch abbindenden Stoff, insbesondere Beton, umschlossen werden soll. Zur Befestigung des mindestens einen Hüllrohrs 2 kann mindestens eine Hilfskonstruktion 1 und/oder mindestens eine tragende Konstruktion in der zu errichtenden Betonstruktur verwendet werden.

In einer Ausführungsform ist das mindestens eine Hüllrohr 2 an einem Ende offen und am anderen Ende verschlossen. Die Hüllrohre 2 können untereinander Kontakt haben oder voneinander beabstandet sein.

Der mindestens eine Glasfasermesssensor 4 wird vor, nach oder während des Einbringens des hydraulisch abbindenden Stoffes zur Herstellung der Betonstruktur in das offene Ende des mindestens einen Hüllrohrs 2 eingebracht, beispielsweise eingeschoben oder eingeblasen, beispielsweise über die ganze Länge des Hüllrohrs 2 bis zu dessen geschlossenem Ende. Vor dem Einbringen des hydraulisch abbindenden Stoffes kann das mindestens eine Hüllrohr 2, in dem der Glasfasermesssensor 4 bereits angeordnet ist, mit einer Flüssigkeit, beispielsweise Wasser, oder mit einem Gel oder mit einer Suspension, beispielsweise einer Zementschlempe, befüllt werden. Dadurch wird das Einlaufen des hydraulisch abbindenden Stoffes in das Hüllrohr 2 vermieden. Die Verwendung eines Gels behindert die Konvektion innerhalb des Hüllrohrs 2, so dass die Ortsauflösung einer Temperaturmessung mittels des Glasfasermesssensors 4 genauer wird.

Insbesondere ist der im Hüllrohr 2 installierte Glasfasermesssensor 4 nicht als Schleife ausgebildet oder gefaltet oder auf sich selbst gedoppelt sondern verläuft einfach entlang des Hüllrohrs 2, beispielsweise gerade. Ebenso kann das Hüllrohr 2 Biegungen aufweisen, denen der Glasfasermesssensor 4 folgt.

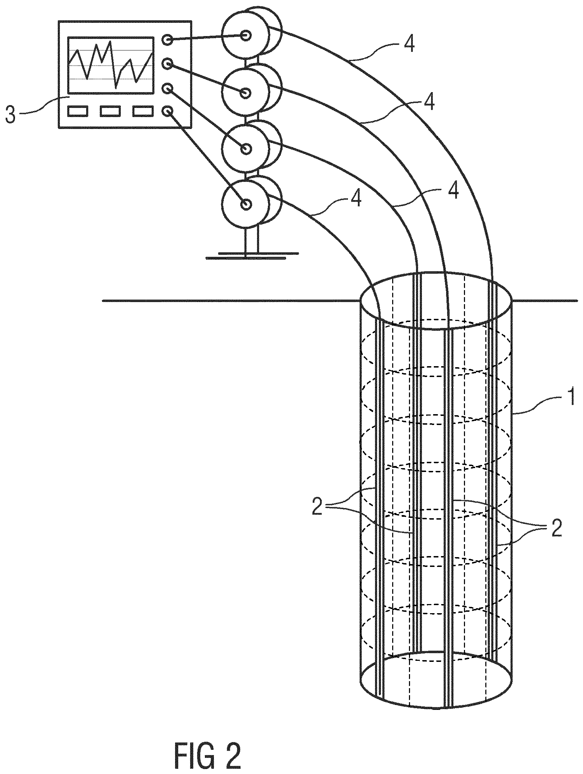

**Figur 2** ist eine schematische Ansicht der Hilfskonstruktion 1, der Hüllrohre 2, des Abfragegeräts 3 und der Glasfasermesssensoren 4, die jeweils in eines der Hüllrohre 2 eingeschoben sind.

Anschließend kann das mindestens eine Abfragegerät 3 zur Erfassung von Temperaturdaten entlang der Glasfasermesssensoren 4 verwendet werden, beispielsweise mittels insbesondere verteilter faseroptischer Temperaturmessung, insbesondere Einbringen von elektromagnetischer Strahlung des optischen, IR- oder UV- Bereichs in die Glasfasermesssensoren 4 und Auswertung rückgestreuter Strahlung.

Ferner kann im Abfragegerät 3 eine Analyse der Temperaturdaten zur Bestimmung der Homogenität des hydraulisch abbindenden Stoffes erfolgen. Die Glasfasermesssensoren 4 können nach Beendigung der Messungen aus den Hüllrohren 2 gezogen und wiederverwendet werden.

Das beschriebene Verfahren vereinfacht den Einbauprozess der Glasfasermesssensoren 4 aufgrund der Hüllrohre 2 wesentlich, liefert eine baustellentaugliche Messeinrichtung und ermöglicht die Wiederverwendung der Glasfasermesssensoren 4. Dadurch kann das Verfahren wesentlich kostengünstiger ausgeführt werden. Durch die Wiederverwendung der Glasfasermesssensoren 4 kann eine Neukalibrierung beim nächsten Einsatz entfallen.

Die Betonstruktur kann beispielsweise eine unterirdische Betonstruktur sein, insbesondere ein Bohrpfahl, eine Schlitzwand oder ein Fundamentkörper. Ebenso kann die Betonstruktur eine oberirdische Betonstruktur sein.

In einer Ausführungsform kann das mindestens eine Hüllrohr 2 perforiert sein, so dass der hydraulisch abbindende Stoff in das Hüllrohr 2 eindringen kann. Auf diese Weise verbessert sich der Wärmeübergang vom hydraulisch abbindenden Stoff auf die Glasfasermesssensoren 4.

Das Verfahren ist nicht nur für die thermische Integritätsprüfung hydraulisch abbindender Stoffe, sondern für jegliche Stoffe, die unter Temperaturänderung, beispielsweise Wärmeabgabe oder Wärmeaufnahme, abbinden, geeignet, beispielsweise für Polymere. Weiter eignet sich das Verfahren für exotherm aushärtende Werkstoffe und/oder endotherm abbindende Werkstoffe.

### BEZUGSZEICHENLISTE

- 1: Hilfskonstruktion
- 2: Hüllrohr
- 3: Abfragegerät
- 4: Glasfasermesssensor

## Patentansprüche

1. Verfahren zur thermischen Überwachung einer Struktur aus einem unter Temperaturänderung abbindenden Stoff, wobei:
- eine Hilfskonstruktion (1) und/oder eine tragende Konstruktion mit mindestens einem daran angeordneten Hüllrohr (2) installiert wird,
- mindestens ein Glasfasermesssensor (4), dessen eines Ende mit einem Abfragegerät (3) verbunden ist oder wird und dessen anderes Ende offen ist, in das mindestens eine Hüllrohr (2) eingebracht wird, derart, dass das offene Ende sich im Hüllrohr (2) befindet,
- der unter Temperaturänderung abbindende Stoff in die Hilfskonstruktion (1) und/oder tragende Konstruktion eingebracht wird, und
- vom Abfragegerät (3) mittels faseroptischer Temperaturmessung eine Temperaturentwicklung entlang des Glasfasermesssensors (4) während des Abbindens erfasst wird.

2. Verfahren nach Anspruch 1, wobei im Abfragegerät (3) ferner eine Analyse der Temperaturentwicklung zur Bestimmung der Homogenität des unter Wärmeabgabe abbindenden Stoffes erfolgt.

3. Verfahren nach Anspruch 1 oder 2, wobei der mindestens eine Glasfasermesssensor (4) nach Beendigung der Temperaturmessung aus dem mindestens einen Hüllrohr (2) gezogen wird.

4. Verfahren nach einem der vorhergehenden Ansprüche,
wobei als unter Temperaturänderung abbindender Stoff ein hydraulisch abbindender Stoff oder ein Polymer verwendet wird.

5. Verfahren nach einem der vorhergehenden Ansprüche, wobei mindestens zwei Hüllrohre (2) so angeordnet werden, dass sie untereinander Kontakt haben oder voneinander beabstandet sind.

6. Verfahren nach einem der vorhergehenden Ansprüche, wobei der mindestens eine Glasfasermesssensor (4) vor, nach oder während des Einbringens des unter Temperaturänderung abbindenden Stoffes zur Herstellung der Struktur in das offene Ende des mindestens einen Hüllrohrs (2) eingebracht wird.

7. Verfahren nach einem der vorhergehenden Ansprüche, wobei der mindestens eine Glasfasermesssensor (4) in das mindestens eine Hüllrohr (2) eingeschoben oder eingeblasen wird.

8. Verfahren nach einem der vorhergehenden Ansprüche, wobei der mindestens eine Glasfasermesssensor (4) über die ganze Länge des Hüllrohrs (2) bis zu dessen geschlossenem Ende eingebracht wird.

9. Verfahren nach einem der vorhergehenden Ansprüche, wobei das mindestens eine Hüllrohr (2), in dem der Glasfasermesssensor (4) bereits angeordnet ist, vor dem Einbringen des unter Temperaturänderung abbindenden Stoffes mit einer Flüssigkeit oder mit einem Gel oder mit einer Suspension befüllt wird.

10. Vorrichtung zur thermischen Überwachung einer Struktur aus einem unter Temperaturänderung abbindenden Stoff, insbesondere mittels des Verfahrens nach einem der vorhergehenden Ansprüche, wobei die Vorrichtung Folgendes umfasst:
- mindestens ein Hüllrohr (2), das an einer Hilfskonstruktion (1) und/oder an einer tragenden Konstruktion angeordnet oder anordbar ist,
- mindestens ein Abfragegerät (3),
- mindestens einen Glasfasermesssensor (4), dessen eines Ende mit dem mindestens einen Abfragegerät (3) verbunden oder verbindbar ist und dessen anderes Ende offen ist,
- wobei das Abfragegerät (3) zur faseroptischen Temperaturmessung einer Temperaturentwicklung entlang des Glasfasermesssensors (4) konfiguriert ist.

11. Vorrichtung nach Anspruch 10, wobei das mindestens eine Hüllrohr (2) an einem Ende offen und an einem anderen Ende verschlossen ist.

12. Vorrichtung nach Anspruch 10 oder 11, wobei das mindestens eine Hüllrohr (2) perforiert oder seitlich geschlitzt ist.
